Europäisches Patentamt

(19) European Patent Office

Office européen des brevets

(11) Publication number: **0 108 564**
**B1**

(12) **EUROPEAN PATENT SPECIFICATION**

(45) Date of publication of patent specification: **04.05.88**

(21) Application number: **83306548.5**

(22) Date of filing: **27.10.83**

(51) Int. Cl.⁴: **G 01 N 33/531,**
G 01 N 33/574, G 01 N 33/58,
A 61 K 39/00, C 12 N 5/00,
C 12 N 11/08, C 12 N 15/00,
C 07 K 7/06, C 07 K 7/08

(54) **Methods for oncogenic detection.**

(30) Priority: **04.11.82 US 439252**
**19.05.83 US 496027**

(43) Date of publication of application:
**16.05.84 Bulletin 84/20**

(45) Publication of the grant of the patent:
**04.05.88 Bulletin 88/18**

(84) Designated Contracting States:
**AT BE CH DE FR GB IT LI LU NL SE**

(56) References cited:
**EP-A-0 036 776**
**EP-A-0 051 873**
**EP-A-0 076 489**
**GB-A-2 041 941**
**US-A-4 217 417**

**SCIENTIFIC AMERICAN, vol. 246, March 1982,
no. 3 J.M. BISHOP "Oncogenes", pages 69-78**
**THE NEW ENGLAND JOURNAL OF MEDICINE,
vol. 303, September 1980, no. 12 J.M. BISHOP
"The Molecular Biology of RNA Tumor Viruses:
A Physician's Guide", pages 675-682**

(73) Proprietor: **THE REGENTS OF THE UNIVERSITY
OF CALIFORNIA**
**2199 Addison Street**
**Berkeley California 94720 (US)**

(72) Inventor: **Cline, Martin J.**
**441 Alma Real Drive**
**Pacific Palisades California, 90272 (US)**
Inventor: **Slamon, Dennis J.**
**23300 Victory Boulevard**
**Woodland Hills California 91367 (US)**

(74) Representative: **Ruffles, Graham Keith et al**
**MARKS & CLERK 57-60 Lincoln's Inn Fields**
**London WC2A 3LS (GB)**

(56) References cited:
**THE LANCET, vol. II for 1982, no. 8288 "Human
Oncogenes", pages 195, 196**
**SCIENCE, vol. 216, May 1982, no. 4548 H.E.
VARMUS "Form and Function of Retroviral
Proviruses", pages 812-820**

Courier Press, Leamington Spa, England.

0 108 564

(58) References cited:

SCIENCE, vol. 217, September 1982, no. 5463
NOBUO TSUCHIDA "Nucleotide Sequence of
the Oncogene Encoding the p21 Transforming
Protein of Kirsten Murine Sarcoma Virus",
pages 937, 938

SCIENCE, vol. 217, September 1982, no. 4563
RAVI DHAR "Nucleotide Sequence of the p21
Transforming Protein of Harvey Murine
Sarcoma Virus", pages 934-936
J. Virol 1979 p- 1196-1203
Virology 84 (1978) p. 429-433

### Description

The mechanism for malignancy of mammalian cells has been and continues to be the subject matter of intense investigation. One of the areas which is considered to be promising in the elucidation of the mechanism is the area of oncogenes. While the occurrence of oncogenes was first detected with retroviruses, it now seems reasonably firm that the viral oncogenes have cellular counterparts. The role of the cellular counterparts is not clear. An excellent review of oncogenes, their properties and particularly the *src* gene may be found in the article by J. Michael Bishop, Scientific American, March 1982:69—78. The article also provides a list of various viral oncogenes, demonstrating that a number of them are involved with phosphorylation.

The *src* gene is found to be not only active in the malignant cell of chickens, but also in the normal cell. The difference appears to be one of degree, rather than of kind, in that the enzyme expressed by the *src* gene would appear to be of much higher concentration in the malignant cell as compared to the normal cell.

In order to be able to determine the presence of a tumor cell, it is necessary to be able to distinguish between normal cells and tumor cells. Therefore, the observed property which is to be diagnostic of the tumor cell must be capable of differentiation from a normal cell or from a physiologic fluid of a normal host, where the fluid rather than cells are assayed. Furthermore, the property should not be specific for the individual, but be common to the malignant nature of the cell.

In both diagnosis and treatment, the opportunity for specifically detecting malignant cells is very important. Any technique, in a high percentage of situations where malignancy is suspected, should be able to distinguish malignant cells from normal cells. Furthermore, the diagnostic technique should be useful for a large number of members of the population and not specific for one or a few members of the population.

Because a cancer cell is derived from a normal cell, most of the properties and components of the malignant cell are the same as the normal cell. Furthermore, there is an increasing view that malignancy is a result of a natural process, which in a certain context results in malignancy. In view of the fact that malignancy may be based on normal processes, which at the time in question have an aberrant result, it is not surprising that there has been substantial difficulty in demonstrating observable differences between normal cells and cancer cells over a broad spectrum of allogeneic hosts.

The following papers provide a general description of oncogenes and the role of retroviruses in tumorigenesis: Bishop, Scientific American, *supra*; Bishop, New England J. of Med. (1980) 303:675—681; Lancet, July 24, 1982, pages 195—196; Cooper, Science (1982) 218:801—806; Varmus, Science (1982) 216:812—820. Papers concerned with specific oncogenes include Becker *et al.*, PNAS USA (1982) 79:3315—3319; Tasuchida *et al.*, Science (1982) 217:937—938 and Dhar *et al.*, *ibid.*, (1982) 217:934—936.

The present application is concerned with a method for evaluating the existence of cellular malignancy in a human, host, said method being characterized in that it comprises: bringing into close association (1) a probe having specific binding affinity for a cellular product, said cellular product being mRNA on its expression product, where said mRNA is complementary to a DNA sequence of a retrovirus capable of transforming a normal cell to malignancy, and

(2) a sample from said human host suspected of containing said cellular product and determining the level of said cellular product by measuring the binding of said probe to said cellular product as indicative of the presence of cellular malignancy.

Compositions are also provided for identifying and treating malignant cells of fresh tumors in a human host. From knowledge of DNA sequences capable of transforming cells of a lower vertebrate to malignancy, polynucleotide probes can be made for determining the level of transcription of said DNA in human cells and receptors produced capable of specifically recognizing determinant sites of peptide products of said DNA sequence. The probes and receptors may be labeled with a wide variety of labels for diagnosis and treatment.

In accordance with the subject invention, novel compositions are provided for the diagnosis and treatment of cancer in humans and other primates. It has now been observed that DNA which is capable of transforming cells of lower vertebrates to malignancy is present in human cells and has a much higher level of transcription and expression in malignant cells than in normal cells. Thus, by being able to detect the higher level of messenger RNA or the expression product of such messenger RNA, the presence of malignant cells in a host may be diagnosed. In addition, the production of the higher level of peptides in the malignant cells can be a basis for treatment of the malignancy. Where the polypeptide expression product can be found in physiological fluids, such as blood, and the levels of the expression product are substantially different in the presence and absence of malignancy, the physiological fluid may be screened as diagnostic for the presence of a particular tumor.

The subject invention provides compositions for evaluating the probability or presence of malignant cells in a group of cells, particularly human cells *in vivo* or freshly removed from a human host. The method using such compositions looks to cellular products such as mRNA or its expression product as diagnostic of the probable presence of malignant cells. The mRNA which is selected for detection will usually be selected as a result of there being RNA present in a retrovirus genome, which retrovirus is capable of

**0 108 564**

transforming mammalian cells to malignancy. Furthermore, the RNA in the retrovirus which is selected is a sequence which does not encode an essential function of the retrovirus and, in fact, may be silent.

The method involves as a first step defining a DNA sequence capable of causing malignancy in a mammalian cell. Once the DNA sequence is defined, polynucleotide sequences can be provided which may serve as probes for detection of elevated levels of messenger RNA to determine whether a cell is malignant. The sequence can also be used for defining polypeptide sequences which can define complementary receptors having high specificity for the peptide sequence. The receptors may then be used for determining the presence or the concentration of the peptide in cells or physiological fluids and for treatment where the receptors can be directed to malignant cells. Also, knowing the nature of the peptide and its function, other means may be available for controlling the elevated production of the particular peptide.

The first step in the subject method is to define the DNA sequence. Various methods can be used for defining the DNA sequence of a retroviral oncogene. For example, retroviruses have been found capable of transforming lower vertebrate cells to malignancy. The retroviruses which have been characterized have been shown to carry DNA sequences comparable to wild type genes present in the host, genes which are now referred to as oncogenes. Furthermore, in the case of Rous sarcoma virus, the expression product of the gene has been isolated and characterized and shown to be kinase. In the case of this kinase, it has also been shown that the kinase is normally produced by the cell, but at a much lower level than when the *src* gene from the Rous sarcoma virus is introduced. A number of viral oncogenes have already been detected in a variety of vertebrates, and the following is a list of the oncogenes and their species of origin.

TABLE 1

| Oncogene | Species of origin |
|---|---|
| v-src | chicken |
| v-fps | " |
| v-yes | " |
| v-fos | " |
| v-myc | " |
| v-erb | " |
| v-myb | " |
| v-rel | turkey |
| v-mos | mouse |
| v-bas | " |
| v-abl | " |
| v-ras | rat |
| v-fes | cat |
| v-fms | " |
| v-sis | monkey |

Other sources of DNA sequences capable of inducing malignant transformations in vertebrate cells may include isolated DNA from a malignant cell or cell line, cloned DNA from a genomic library or cloned DNA from a messenger RNA library, where the total messenger of the malignant cell is reverse transcribed to DNA and cloned. Either of these libraries may be screened for their ability to induce malignancy. A refinement in the technique of screening may be achieved by taking the total messenger from a normal cell and preparing cDNA from the messenger. One can then use the single stranded DNA as a probe to remove messenger RNA associated with the normal cell from the total messenger RNA from a malignant cell. The residual messenger RNA will then include messenger being expressed by genes associated with the malignancy. One may then use the messenger to screen a genomic library and use the cloned DNA which hybridizes with messenger in a bioassay for the determination of the ability to transform to malignancy. Other ways will also become available in time for detecting and defining DNA sequences capable of transforming normal cells.

A further analysis can be employed by screening cDNA from fetuses with messenger RNA from malignant cells. Particularly, where the oncogene is a gene which is silent or relatively quiescent in the nature vertebrate, while highly active in the embryo, the screening may further serve to narrow the field of sequences to be screened.

Once having identified a DNA sequence capable of inducing malignancy, a cloned viral oncogene or short polynucleotide sequences can be employed as probes for detection of the level of production of messenger RNA in cells suspected of being malignant. The preparation of both RNA and DNA nucleotide sequences, the labeling of the sequences, and the preferred size of the sequences has received ample description and exemplification in the literature. Normally, a sequence should have at least about 14 nucleotides, usually at least about 18 nucleotides, and the polynucleotide probes may be one or more kilobases. Various labels may be employed, most commonly radionuclides, particularly $^{32}P$. However, other techniques may also be employed, such as using biotin modified nucleotides for introduction into a

4

# 0 108 564

polynucleotide. The biotin then serves as the site for binding to avidin or antibodies, which may be labeled with a wide variety of labels, such as radionuclides, fluorescers or enzymes. Alternatively, antibodies may be employed which can recognize specific duplexes, including DNA-protein duplexes. The antibodies in turn may be labeled and the assay may be carried out where the duplex is bound to a surface, so that upon the formation of duplex on the surface, the presence of antibody bound to the duplex can be detected.

By isolating the nucleotide sequence for the whole oncogene, the sequence of bases may be determined by known means, e.g. Maxam and Gilbert, PNAS USA (1977) 74: 560. The sequence can be used for the determination of the amino acid sequence of the protein expressed by the oncogene. By identifying codons for methionine followed by a sequence which does not have stop codons which prevent expression, one can usually find a single sequence in frame with a methionine codon for defining the oncogene.

Alternatively, hybrid DNA technology may be employed for obtaining expression. The DNA sequence may be restriction mapped and appropriate sites for cleavage defined. In this way, the sequence may be excised and introduced into a vector having the appropriate regulatory signals. After obtaining expression of the DNA sequence, antibodies can be made to the polypeptide. By employing oocytes for expression of the messenger RNA which is then translated to produce the peptide expressed by the oncogene, the protein defined by the messenger may be produced. The identity of the peptide from the oocyte which the peptide produced by the expression of the hybrid DNA may then be determined.

Once the protein has been identified and verified, one can then use the protein or subunit peptides as an antigen for the production of antibodies for diagnosis and treatment. Antibodies can be prepared in a variety of ways, depending upon whether monoclonal or polyclonal antibodies are desired. For polyclonal antibodies, a vertebrate, normally a domestic animal, is hyperimmunized with the antigen and blood collected shortly after repeat immunizations and the gamma globulin isolated. For monoclonal antibodies, a small animal is hyperimmunized, the spleen removed and the lymphocytes fused with an appropriate fusing partner. The resulting hybridomas are then grown under limiting dilution and clones providing the desired antibodies selected.

Rather than preparing the entire peptide, one can determine various regions which are likely to be determinant sites and use these oligopeptides of at least eight amino acids, usually at least 10 and not more than 20, usually not more than 18 amino acids, to define a hapten which can be used to induce antibody formation. The oligopeptide is bound to an appropriate immunogen and introduced into a vertebrate to produce antibodies, either polyclonal or monoclonal antibodies, as described previously.

Accordingly, the present invention also provides as defined in claim 17 a series of oligopeptides corresponding to antigenic regions in the peptide expression products of RNA present in retrovirus oncogenes. Exemplary species of the antigenic oligopeptides useful in accordance with the subject invention are listed below in groups based on the retroviral oncogene (expression product) which is recognized by antibodies produced from the oligopeptide.

A. Myb

met-ala-phe-ala-his-asn-pro-pro-ala-gly-pro-leu-pro-gly-ala
pro-phe-his-lys-asp-gln-thr-phe-thr-glu-tyr-arg-lys-met-his-gly-gly-ala-val
pro-phe-his-lys-asp-gln-thr-phe-thr-glu-tyr-arg-lys-met
asp-asn-thr-arg-thr-ser-gly-asp-asn-ala-pro-val-ser-cys-leu-gly-glu

B. Src

arg-leu-ileu-glu-asp-asn-glu-tyr-thr-ala-arg-gln-gly-ala-lys-phe-pro
trp-arg-arg-asp-pro-glu-glu-arg-pro-thr

C. Ras$^{Ki}$

arg-leu-lys-lys-ileu-ser-lys-glu-glu-lys-thr-pro-gly-cys-val-lys-ileu-lys-lys
asp-leu-pro-ser-arg-thr-val-asp-thr-lys-gln-ala-gln-glu-leu-ala-arg
met-thr-glu-tyr-lys-leu-val-val-gly-ala-ser-gly-val-gly-lys-ser-ala

D. Ras$^{Ha}$

val-arg-glu-ileu-arg-gln-his-lys-leu-arg-lys-leu-asn-pro-pro-asp-glu-ser-gly-pro
met-thr-glu-tyr-lys-leu-val-val-val-gly-ala-gly-gly-val-gly-lys-ser-ala
val-asp-glu-tyr-asp-pro-thr-ileu-glu-asp-ser-tyr-arg-lys-gln-val

E. Fes

arg-his-ser-thr-ser-ser-ser-glu-gln-glu-arg-glu-gly-gly-arg
asn-gln-gln-thr-arg-glu-phe-val-glu-lys-gly-gly-arg
pro-glu-val-gln-lys-pro-leu-his-glu-gln
ala-ser-pro-tyr-pro-asn-leu-ser-asn-gln-thr-arg

F. Myc

arg-leu-ileu-ala-glu-lys-glu-gln-leu-arg-arg-arg-glu-gln
asn-asn-glu-lys-ala-pro-lys-val-val

5

Since additional amino acid residues can be added to one or both ends of each of the oligomeric peptide sequences given under subparagraphs A through F above without changing the antigenic character of the resulting peptide, this invention also contemplates a series of antigenic oligopeptides of up to 50, and preferably 25, amino acids containing at least one of the amino acid sequences listed under A through F above.

The antigenic oligopeptides, according to the invention containing one or more of the amino acid sequences given in subparagraphs A through F above, can be prepared by synthetic techniques, and by techniques employing hybrid DNA technology. For those polypeptides and oligopeptides of the invention containing up to about 50 amino acid residues, conventional solid phase peptide synthesis is suitably employed. This general synthetic procedure for making peptides, which is described, for example, in U.S. Patent 4,341,761 to Canfield et al., employs known side-chain protecting groups and conventional supports—e.g. polystyrene resin supports such as chloromethylated resins, hydroxymethyl resins or benzhydrylamine resins—to affect the amino acid coupling. In techniques utilizing recombinant or hybrid DNA technology, the oligopeptides according to the invention containing up to, for example, 20 amino acids can be used to deduce the codon sequence for the nucleotide (DNA) which codes for the oligopeptide in question. Using this codon sequence as a template, double stranded DNA can then be synthesized using known techniques and inserted into a vector DNA or cloning vehicle such as E. Coli plasmid. Transformation of a suitable host, for example a microorganism, such as E. Coli or, for instance, other cell lines with the recombinant vector, affords a means to obtain expression of the desired oligopeptide.

In those situations where the human gene is different form the v-onc, e.g. human c-ras, the above described techniques may be used for isolating the gene, mRNA or pseudo-gene and obtaining antibodies to the human expression product. The human oncogene would be expected to have substantial complementarity to the related v-onc, normally differing in fewer than about 5% of the basis, generally differing by fewer than 5% of the amino acids in the expression product.

The antibodies may be used in a variety of ways. Particularly, they may be used for diagnosis. In instances where the antigen may be found in a physiological fluid at an elevated concentration only when malignancy exists, the physiological fluid, such as serum, plasma, whole blood or cerebrospinal fluid may be assayed. Antibodies employed in assays may be labeled or unlabeled. Unlabeled antibodies may be employed in agglutination; labeled antibodies may be employed in a wide variety of assays, employing a wide variety of labels, such as radionuclides, enzymes, fluorescers, enzyme substrates or cofactors, or the like. These techniques are amply defined in the literature and exemplary assays may be found in U.S. Patent Nos. 3,817,834, 3,935,074, 4,233,402 and 4,318,980, as illustrative.

In some techniques, it will be useful to label the antigen or fragment thereof, rather than the antibody and have a competition between labeled antigen and antigen in the sample for antibody. In this situation, it is common to provide kits which have the combination of the labeled antigen or labeled fragment and the antibody in amounts which provide for optimum sensitivity and accuracy.

In other situations, it is desirable to have a solid support, where either antigen or antibody is bound. A polyepitopic antigen can serve as a bridge between antibody bound to a support and labeled antibody in the assay medium. Alternatively, one may have a competition between labeled antigen and any antigen in the sample for a limited amount of antibody.

Where the antigen may not be found in a physiological fluid or if found there is not diagnostic of malignancy, then cells will have to be isolated and the cells assayed for the presence of messenger RNA or the antigen. Methods of detecting messenger RNA have already been described. For detecting the antigen, the tissue sample may be lysed by conventional methods, e.g. bases, detergents, cellular debris separated by filtration or centrifugation and the filtrate or supernatant isolated and assayed.

For purposes of therapy, either xenogeneic or allogeneic antibodies may be employed, depending upon the nature of the treatment, and whether the foreign antibodies will induce an immune response. The literature has described a number of ways of making human antibodies, where it is found that mouse or other mammalian antibodies are not satisfactory. The antibodies may be used in a wide variety of ways. By employing the appropriate IgG (other than IgG$_1$), one may induce lysis through the natural complement process. Alternatively, the lysing portion of a toxin may be joined to the antibodies particularly a Fab fragment. The antibodies may be bound to liposomes for directing the liposomes to the malignant cells to become ingested by the cells by merging of the membranes. Other labels may also be bound to the antibodies, such as radionuclides, fluorescers and enzymes. By introducing the antibodies in vivo, the antibodies will direct the label to the malignant cell, where the presence of malignance may be diagnosed or treated.

The formulation of the antibodies will vary widely, depending on the nature of the label, the purpose of the antibodies, the site to which the antibodies are to be directed. Usually, the antibodies will be formulated in a physiologically acceptable carrier, e.g. saline or phosphate buffered saline, and injected into the host, when possible at the desired site, and when this is not possible, into a circulating system, such as blood.

The antibodies obtained in accordance with this invention can also be used to isolate cells expressing the oncogene and to remove cells in vitro from a heterogeneous cell population containing cells expressing the oncogene. Separation can be achieved with a fluorescence activated cell sorter (FACS). This same technique can be used for identifying and isolating cells expressing the oncogene. For removing cells expressing the oncogene from a mixture of cells, the subject antibodies may be combined with

6

complement, joined to the lysing fragment (A fragment) of a toxin (see E.P.O. application No. 17,507 and U.K. Patent Application No. 2,034,324) or the cells agglutinated and separated by physical means.

The following examples are offered by way of illustration and not by way of limitation.

Tumors were obtained from fresh surgical specimens at the time of resection and were untreated by chemotherapy or radiotherapy. An effort was made to obtain only viable tumor and to process the tissues as rapidly as possible to avoid messenger RNA (mRNA) degradation. Specimens were quickly frozen and stored in liquid nitrogen until processed for RNA. When the surgical specimens included wide margins of normal tissue, some of this was taken for analysis as an internal control of the level of c-onc gene expression. C-onc gene expression could then be compared in normal and malignant tissue from the same patient. As little as 20 pg of Maloney murine sarcoma virus equivalent to approximately one RNA transcript of 3 kilobases (kb) per cell or approximately 2 micrograms of poly A RNA applied to the filter could be detected by this method (Kafatos et al., Nucleic Acids Res. (1979)7:1541). By use of appropriate controls including unrelated RNA's, poly A-negative fraction RNA, plasmid DNA, and mouse and human DNA, false-negative as well as false-positive results could reasonably be excluded. The dot blots were quantitatively evaluated by means of a soft laser scanning densitometer. Where sufficient material was available, mRNA was further characterized by Northern analysis to confirm the presence of, and to size, specific transcripts (Thomas, PNAS USA (1980)77:5201).

Expression of 13 cellular oncogenes in 14 tumors was examined by DNA—RNA hybridization techniques. These data are summarized in Table 2.

TABLE 2
Human malignancy*

| V-onc probe | Gastrointestinal malignancies | | | | Renal cell carcinoma | | | | Ovarian carcinoma | | | Lung adenocarcinoma | | Lymphosarcoma |
| | Colon | | Adenocarcinoma of Small bowel | Rectum | | | | | | | | | | |
| | 1 | 2 | 1 | 1 | 1 | 2 | 3 | 4 | 1 | 2 | 3 | 1 | 2 | 1 |
|---|---|---|---|---|---|---|---|---|---|---|---|---|---|---|
| Myc** | +++ | +++ | +++ | +++ | ++++ | ++++ | ++ | ++++ | ++ | ++ | ++ | ++ | ++ | — |
| Myb | — | — | — | — | — | — | — | — | — | — | — | — | ++ | — |
| Erb | — | — | — | — | — | — | — | — | — | — | — | — | — | — |
| Src | — | — | — | — | — | — | — | — | — | — | — | — | — | +++ |
| Yes | — | — | — | — | — | — | — | — | — | — | — | — | — | — |
| Abl*** | — | — | — | — | — | — | — | — | — | — | — | — | — | — |
| Fos | +++ | +++ | +++ | +++ | ++ | +++ | +++ | ++ | + | ++ | ++++ | + | ++ | — |
| Mos | — | — | — | — | — | — | — | — | — | — | — | — | — | — |
| Ras$^{Ha}$ | ++ | ++ | ++ | ++ | ++ | ++ | ++ | ++ | ++ | ++ | ++ | ++ | ++ | — |
| Ras$^{KI}$ | ++ | ++ | ++ | ++ | ++ | ++ | ++ | ++ | ++ | ++ | ++ | ++ | ++ | — |
| Fes | — | — | — | — | — | — | — | — | — | — | — | + | ++ | — |
| Fms | — | — | — | — | — | — | — | — | — | — | — | — | — | — |
| Sis | — | — | — | — | — | — | — | — | — | — | — | — | — | — |

*Increasing numbers of pluses indicates increasing intensity of hybridization of tissue mRNA to v-onc probes.
**Avian
***Murine

**0 108 564**

Three patterns were observed: 1) expression of specific c-onc mRNA sequences in all or nearly all tumor samples (e.g. c-myc); 2) detection of c-onc expression in sporadic tumors (e.g., c-fes); and 3) no detectable expression (e.g., c-mos).

No significant expression or mRNA sequences homologous to c-erb, c-yes, c-abl, c-mos, c-fms, or c-sis could be detected. This was not the result of lack of homology between the viral gene probe and the human messenger RNA, since it was possible to detect homologues of all these probes in human genomic DNA. RNA from microscopically normal tissue did not contain any detectable transcripts by this analysis.

Four cellular oncogenes showed a consistent pattern of expression in a variety of human tumors. These were c-myc, c-fos, c-ras$^{Ha}$, and c-ras$^{Ki}$. A comparison was made of the intensity of hybridization, which was possible since all probes were labeled to approximately the same specific activity. V-myc and v-fos demonstrated the highest intensity of hybridization to human tumor RNA's, suggesting a large number of copies of mRNA per cell. Expression of both these genes was observed in all malignancies examined, C-ras$^{Ha}$, and c-ras$^{Ki}$ sequences were also detected in most of the human tumors but with less intense hybridization.

Messenger RNA sequences related to c-fes were detected in only 2 of 14 tumors examined, both of these were lung cancers.

C-myb expression was detected in only one of 14 tumors, this, too, was a lung cancer.

C-src messenger RNA sequences were observed only in circulating tumor cells of a patient with lymphosarcoma.

In order to test whether expression of cellular oncogene sequences was related to neoplasia, an effort was made to obtain both grossly normal-appearing tissue and obviously malignant tissue from the same site in the same patient at the same time. Hybridization studies were then performed on RNA samples from the tumor and from adjacent non-involved tissues. In 6 of the 14 patients it was possible to perform this analysis. In 1 of these 6 cases the presumed normal tissue was subsequently shown by histologic analysis to be infiltrated by tumor. In 4 of the remaining 5 cases there was differential expression between the tumor and normal tissue, with low or undetectable levels observed in the normal tissues and elevated levels observed in the malignancy. Three of the renal cell carcinomas and one colon carcinoma demonstrated this phenomenon.

Blot analysis of RNA from cells in the areas of the tumor sample and the control sample showed a correlation between the presence or absence of tumor and c-onc gene expression. In one tumor, an adenocarcinoma of the small bowel, c-onc-related sequences were found in histologically normal adjacent tissue.

Analyses of poly A RNA from tumors and control tissues were performed by the Northern technique. Two c-myc-related transcripts of 4.0 and 2.0 kb were found in all tumors examined. In addition to these transcripts, there was obvious degradation of some of the messenger RNA in these hybridization analyses, most likely resulting from degradation occurring during tissue anoxia in the period after surgical removal of the tissue.

Using the procedures given above, several other tumor types obtained from fresh surgical specimens were examined for c-onc gene expression. In this series of tests, DNA—RNA hybridization was used to look for expression of 10 different cellular oncogenes in 9 tumors. The data obtained are summarized in Table 3 below.

## TABLE 3
### Human malignancy*

| V-onc probe | Breast carcinoma | | | Uterine carcinoma | Thymoma | Hodgkin's | Non-Hodgkin's lymphoma | Chronic myelocytic leukemia | |
|---|---|---|---|---|---|---|---|---|---|
| | 1 | 2 | 3 | 1 | 1 | 1 | 1 | 1 | 2 |
| Myc** | ++ | +++ | +++ | — | + | ++ | + | ++ | — |
| Myb | + | ++ | — | — | — | + | — | + | — |
| Src | + | + | — | — | — | ++ | + | +++ | — |
| Rel | — | — | — | — | — | — | — | — | — |
| Abl*** | — | — | — | — | — | — | — | — | — |
| Fos | ++ | ++ | ++ | — | + | ++ | + | ++++ | ++++ |
| Ras$^{Ha}$ | +++ | +++ | +++ | — | + | ++ | + | ++ | + |
| Ras$^{KI}$ | + | + | ++ | — | — | + | — | + | + |
| Fes | + | ++ | +++ | — | — | + | + | +++ | ++ |
| Sis | — | — | — | — | — | — | — | — | — |

*Increasing numbers of pluses indicates increasing intensity of hybridization of tissue mRNA to v-onc probes.
**Avian
***Murine

The results given in Table 3 correlate fairly well with the results previously reported in Table 2 in that the cellular oncogenes c-myc, c-fos, c-ras$^{Ha}$, and c-ras$^{KI}$ show a consistent pattern of expression in the additional tumor types examined. Further, c-myb, c-src and c-fes were also detected in several additional tumor types whereas c-rel, c-abl and c-sis expression was not observed in any of the additional tumor types examined. Interestingly, none of the cellular oncogenes looked for were found to be expressed at any significant level in the single uterine carcinoma evaluated.

To determine whether the messenger RNA shown to be present in malignant cells in elevated amounts were related to genes involved in embryogenesis, experiments were carried out generally as follows. Total RNA was isolated from embryo/fetuses of random-bred Swiss mice at daily intervals starting on the 6th day of gestation (day of coital plug was taken as day 0 of prenatal development). Beginning at day 10 of prenatal development, the embryo proper was separated from the extraembryonal membranes and placenta. The small size prior to day 10 prevented separation and therefore the embryos of days 6—9 represent the entire conceptus as dissected from the uterine wall.

Aliquots of poly(A)-containing RNA (poly(A$^+$)RNA), were isolated by affinity chromatography on oligo(dT)-cellulose columns and spotted on nitrocellulose paper (dot blots) (Kafatos et al., Nucl. Acids Res. (1979) 7:1541—1552). The samples were hybridized to [$^{32}$P]-labeled molecularly cloned oncogene-specific probes. Dot blots were quantitatively evaluated by means of a soft laser scanning densitometer. Transcriptional activity of c-onc's was additionally studied in more detail in various tissues of newborn and 10 day old mice. Agarose gel electrophoresis followed by blotting on nitrocellulose paper (Northern blotting), (Thomas, PNAS USA (1980) 77:5201—5205) was used to confirm the results obtained by dot blot analysis and additionally to determine the sizes of the different c-onc-related transcripts.

More specifically, RNA was isolated from Swiss-Webster mouse embryo fetuses at various stages of development using the guanidine thiocyanate method. (Cox, Methods Enzymol), (1967) 12:120—129; Adams et al., PNAS USA (1980) 74:3399—3043).

As indicated above, days 6—9 Swiss-Webster mouse embryos represent the entire conceptuses including all extraembryonal tissues, such as membranes and those cells giving rise to the placenta at later developmental stages. At all later stages, the embryo proper was dissected free of extraembryonal tissues. RNA was selected for (poly(A$^+$)-RNA by one cycle of chromatography on oligo(dT)-cellulose columns (Aviv and Leder, PNAS USA (1972) 69:1408—1412). (Poly(A$^+$) RNA was dissolved in water, boiled, quick-cooled on ice and 3 µg-(1.5 µl) were applied to sheets of nitrocellulose paper which had previously been equilibrated with 20×SSC (1×SSC is 0.15 NaCl, 0.015M sodium citrate) and air dried. After baking overnight at 80°C, the blots were prehybridized for at least 4h at 45°C in a buffer containing 0.75M NaCl, 0.05M sodium phosphate (pH7.5), 0.005M EDTA, 0.2% SDS, 10mg of glycine/ml, 5×Denhardt's reagent (1×Denhardt's reagent is 0.02% each of Ficoll (Registered Trade Mark) bovine serum albumin and polyvinylpyrrolidone), 0.25mg of denaturated herring DNA/ml and 50% formamide.

The blots were hybridized for about 20h at 45°C with $1×10^6$cpm of nick-translated probe/ml of hybridization buffer (prehybridization buffer with Denhardt's reagent at 1×). The cloned oncogene fragments purified from vector sequences by preparative agarose gel electrophoresis were nick-translated (Rigby et al., J. Mol. Biol. (1977) 113:237—251) in the presence of [$^{32}$P]-dCTP $1.184×10^{14}$Bq/mmol (3200Ci/mmol) to specific radioactivities of about $1—2×10^9$cpm/µg of DNA. After hybridization, the blots were washed three times in 1×SSC at 50°C for a total of about 2h and exposed to preflashed X-ray films with intensifying screens at −70°C for 72h.

Employing the above procedure, a number of known oncogenes were screened to determine whether they were expressed in the embryos. The following table indicated the individual oncogene and the observations concerning their expression in embryonic cells.

TABLE 4

| | | | mRNA production embryos | | |
|---|---|---|---|---|---|
| Oncogene | Virus | Disease | days 6—9 | 10—18 | Fetus |
| fos[1] | FBJ-osteosarcoma | osteosarcoma | + | — | + |
| abl[2] | Abelson leukemia | lymphoma | + | + | + |
| ras[Ha3] | Harvey sarcoma | erythroleukemia, sarcoma | + | + | + |
| mos[4] | Malony sarcoma | sarcoma | — | — | — |
| myc[5] | Avian myelocytomatosis | carcinoma, sarcoma luekemia | + | + | |
| erb[6] | Avian erythroblastosis | leukemia, sarcoma sarcoma | — | + | |
| src[7] | Rous sarcoma virus | sarcoma | + | + | |
| myb[8] | Avian myeloblastosis | leukemia | — | — | |
| fes[9] | Snyder-Theilin feline sarcoma | sarcoma | — | — | |
| sis[10] | Simian sarcoma | sarcoma | + | + | |

[1] Curren et al., J. Virol. (1982)
[2] Goff et al., Cell (1980) 22:777—785
[3] Ellis et al., J. Virol. (1980) 36:408—420
[4] Oskarsson et al., Science (1980) 207:1222—1227: Jones et al., PNAS USA (1980) 77:2651—2655
[5] Eva et al., Nature (1982) 295:116
[6] Gonda et al., Mol. Cell. Biol. (1982) 2:617
[7] Wang et al., (1977) J. Virol, 24:64
[8] Vister et al., PNAS USA (1982) 79:3677—3681
[9] Fedele et al., PNAS (1982), in press
[10] Devare et al., PNAS (1982) 79:3179—3182

Relatively high levels of c-fos related sequences were detected in (poly(A$^+$)-RNA prepared from 6, 7, 8 and 9 day conceptuses containing the embryo proper and extra-embryonal tissues. More than 10-fold lower fos expression was observed in embryos of later development stages dissected free of extraembryonal tissues. Data from the placenta and extraembryonal membranes of fetuses from days 10 to 18, showed that expression was primarily in those tissues. In postnatal tissue, c-fos expression could be observed in all tissues investigated with stronger hybridization to the fos-specific probe from bones.

Hybridization showed that for c-abl about three-fold higher levels in the embryo proper than in extraembryonal membranes and placenta is observed at the 10th day of gestation, as compared to the concentration observed in the 6, 7 and 9 day conceptuses. Expression of c-abl in the fetus appears to decrease after the 11th day of prenatal development. The oncogene c-*abl* is transcriptionally active in all postnatal mouse tissues examined with spleen and thymus (poly(A)$^+$ RNA exhibiting a slightly stronger hybridization than from other tissues.

The oncogene c-*ras*$^{Ha}$ was found to be expressed in considerable, but similar levels at all stages of prenatal development both in the embryo proper as well as in extraembryonal tissues. High levels of c-*ras*$^{Ha}$ expression were also observed in various tissues of newborn or 10 day old mice, particularly in bone, brain, kidney, skin and spleen.

The oncogene c-*myc* was detectable at days 7 and 8, but much higher levels were observed in late embryonic development (days 17 and 18).

The oncogene c-*erb* had maximum hybridization at 13 days, while no hybridization was observed at day 6.

The oncogene c-*src* was detected at its highest levels in the latter half of mouse embryonic development with an increase beginning at day 14, peaking at day 15 and gradually decreasing thereafter. For the oncogene c-*sis*, peak expressions were observed at days 7 and 16, the day-7 peak was 1.5 to 3 times higher than all other days and the day-16 peak was 1.5 to 2 times higher than days 9 to 13 and days 17 and 18.

In the next study, the nucleotide sequence of the presumed encogene region of Avian myeloblastosis virus *myb* was employed (Vister et al. (1982) *supra*). Using the published nucleotide sequence, a number of antigenic oligopeptide sequences were derived and seven of the polypeptides so derived were synthesized and evaluated as being potentially antigenic. These seven oligopeptides, which are also listed hereinbefore as antigenic oligopeptides according to the invention, have the following formulas:

(1) pro-phe-his-lys-asp-gln-thr-phe-thr-glu-tyr-arg-lys-met
(2) pro-ser-pro-pro-val-asp-his-gly-cys-leu-pro-glu-glu-ser-ala-ser-pro-ala-arg
(3) asp-asn-thr-arg-thr-ser-gly-asp-asn-ala-pro-val-ser-cys-leu-gly-glu
(4) pro-gln-glu-ser-ser-lys-ala-gly-pro-pro-ser-gly-thr-thr-gly
(5) met-ala-phe-ala-his-asn-pro-pro-ala-gly-pro-leu-pro-gly-ala
(6) pro-pro-val-asp-his-gly-cys-leu-pro-glu-glu-ser-ala-ser-pro-ala
(7) pro-phe-his-lys-asp-gln-thr-phe-thr-glu-tyr-arg-lys-met-his-gly-gly-ala-val

The polypeptides were linked to keyhole limpet hemocyanin in accordance with conventional techniques (Dockray, Regulatory Peptides (1980)1:169) and the resulting immunogen was used to immunize rabbits in a first injection with complete Freund's adjuvant, followed by injections with incomplete Freund's adjuvant over periods of three to four weeks to hyperimmunize the rabbits. The rabbits were bled repeatedly over a period of six months. Of the seven oligopeptides which resulted in the production of antibodies, antibodies to the peptides (5 and 7) were selected for detailed analysis. The antibodies were reacted with radioactively labeled cell lysates from a cell line containing multiple copies of the Avian myeloblastosis virus and with lysates from appropriate noninfected cell lines. Antibody No. 5 identifies a specific protein of approximately 58,000 daltons, which is present in the virus-infected cell line but not in controls.

Antibody against polypeptide 5 was also reacted with the plasma of chickens bearing tumors induced by *amv*. A band similar to that observed with the above lysates of approximately 48,000 daltons was identified.

Antisera to polypeptide No. 5 was also reacted with lysates of a myeloid human leukemia cell line (HL-60) which is known to express messenger RNA transcripts of the c-myb gene (Gallo and Wong-Staal, Blood (1982) 60:545). This antibody reacted with a protein of about 90,000 daltons. In freshly isolated myeloid leukemia cells, the antibody identifies a series of proteins, 14 kd to 70 kd, not present in normal white blood cells.

The polyclonal antibodies to the fragment No. 5 of the *myb* protein was tested for its ability to kill normal and leukemic cells. The procedure employed is described in Terasaki and McClelland, *infra*. The data are set forth in the following table.

### TABLE 5
### Cytotoxicity* of anti-Myb2 antibody against human cells

| | | Cells[++] | | | | | | | | |
|---|---|---|---|---|---|---|---|---|---|---|
| | | Normal | Normal | Molt4 | HL-60 | Common ALL | | AML | T-ALL | |
| Antiserum | Dilution | B | T | (T) | (AML) | 1 | 2 | (5)** | 1 | 2 |
| Medium Alone (− control) | | 1 | 1 | 1 | 1 | 1 | 1 | 1 | 1 | 1 |
| ALS+ (+control) | — | 8 | 8 | 8 | 8 | 8 | 8 | 8 | 8 | 8 |
| Anti-*myb* | — | 1 | 1 | 8 | 6 | 6 | 1 | 6 | 6 | 6 |
| | 1:2 | 1 | 1 | 6 | 1 | 1 | 1 | 6 | 6 | 2 |
| | 1:4 | 1 | 1 | 6 | 1 | 1 | 1 | 4 | 4 | 1 |
| Pre-Immune | — | 1 | 1 | 4 | 1 | 1 | 1 | 1 | 4 | 2 |
| | 1:2 | 1 | 1 | 2 | 1 | 1 | 1 | 1 | 2 | 2 |
| | 1:4 | 1 | 1 | 2 | 1 | 1 | 1 | 1 | 2 | 2 |

*8=80—100% killing 6=60—80% killing 1=0—10% killing
The method of Terasaki and McClelland, Nature (1964)204:998, was employed for complement lysis.
**3 of 5 are killed; a representative one is shown
+ALS—antileukocyte serum
++Molt 4 is a non-alignant human T lymphoid cell line that is known to express *myb* mRNA.
HL-60—human myeloid leukemia cell line
AML—acute myeloid leukemia
ALL—acute lymphocyte leukemia
T-ALL—T-cell acute lymphoblastic leukemia

The above results demonstrate that the expression product of the *myb* gene can react with antibodies to produce lysis with complement. Thus, the *myb* protein appears to be a surface membrane protein which is available for binding to antibodies. By identifying proteins to which specific antibodies will bind, which proteins have diagnostic value as indicative of malignance, the malignant cells can be identified and treated. In the subject work, no determination has been made as to the specificity or cross-reactivity of the subject antibodies. Since only a fragment was used to prepare the antibodies, it would be expected that antibodies of greater binding specificity and avidity could be prepared with the whole protein, particularly with the whole protein in a membrane. The subject antibodies can be used to select for antibodies binding to the same or other determinant site.

The subject data demonstrate that antibodies can be prepared which do not affect normal B- and T-cells, but are cytotoxic in combination with complement for a variety of malignant cells. Therefore, the antibodies can be used for cancer therapy without the hazard of substantially inactivating the immune system.

It is evident from the above results, that one can detect the presence of malignancy in a human host by determining the transcription and/or expression products of the oncogene. One can screen retroviruses or other source of nucleic acids to transform vertebrates to malignancy. One may then use these nucleic acids to deduce peptide composition and screen malignant cells for transcripts or peptides, by hybridization in the former case and with appropriate receptors in the latter case, employing any of a wide variety of diagnostic assays. Antibodies can be produced to the peptides, which antibodies may be labeled and may then be used for diagnosing the presence of a peptide diagnostic of malignancy. The oncogenic proteins are found to be available for binding to antibodies as surface membrane proteins. The antibodies may serve as diagnostic reagents for determining the presence of malignancy and determining the location of malignant cells. The antibodies may also serve in treating tumors *in vivo* by using radionuclides, toxins, in combination with the host complement system or opsonins, or other antibody dependent lytic system. The antibodies find use in pre- and postoperative systems, in the latter determining whether complete removal has occurred, whether metastases exist. The antibodies can be used postoperatively to destroy any remnants of the tumor which may not have been excised.

**0 108 564**

Claims for the Contracting States: BE, CH, DE, FR, GB, IT, LI, LU, NL, SE

1. A method for evaluating the existence of cellular malignancy in a human host, said method being characterized in that it comprises:

bringing into close association (1) a probe having specific binding affinity for a cellular product, said cellular product being mRNA or its expression product, where said mRNA is complementary to a DNA sequence of a retrovirus capable of transforming a normal cell to malignancy, and (2) a sample from said human host suspected of containing said cellular product; and

determining the level of said cellular product by measuring the binding of said probe to said cellular product as indicative of the presence of cellular malignancy.

2. A method according to Claim 1, characterized in that said sample is cells from said human host.

3. A method according to Claim 1, characterized in that said sample is a physiological fluid from said human host.

4. A method according to Claim 1, characterized in that said DNA sequence is selected from the group consisting of the oncogenes *src, fps, yes, fos, myc, erb, myb, rel, mos, bas, abl, ras, fes, fms,* and *sis.*

5. A method according to any of Claims 1, 2, 3 or 4, characterized in that said probe is an antibody.

6. A method according to Claim 5, characterized in that said antibody is labeled with a label capable of providing a detectable signal.

7. A method according to any of Claims 1, 2, 3 or 4, characterized in that said probe is a polynucleotide of at least 14 bases complementary to said mRNA.

8. A method according to claim 1, characterized in that it comprises a method for evaluating the existence of leukemia in a human host, said method comprising:

combining a probe comprising an antibody having specific binding affinity for a cellular product of the oncogene *myb*, and blood cells from a human host suspected of having leukemia; and

detemining the level of said cellular product by measuring the binding of said antibodies to said cellular product as diagnostic of a leukemic host.

9. A method according to Claim 8, characterized in that said antibodies are produced in response to an oligopeptide mimicking a portion of the conformation of the *myb* protein.

10. An *in vitro* method for substantially eliminating human malignant cells from a combination of human malignant and normal cells, characterized in that it comprises:

combining said combination of cells with an antibody specific for an expression product of a DNA sequence present in a retrovirus genome or substantially complementary to said DNA sequence, which sequence is expressed in said malignant cells as a surface protein, said combining being performed under cytotoxic conditions; and

isolating normal cells, substantially free of malignant cells.

11. A method according to Claim 10, characterized in that said separation occurs in the presence of complement as said cytotoxic condition.

12. A method according to Claim 10, characterized in that said antibodies are labeled with a radionuclide as said cytotoxic condition.

13. A method according to any of Claims 10, 11 or 12, characterized in that acid DNA sequence is the myb gene.

14. Antibodies characterized in that they are specific for the expression product of the human oncogenes c-*myc*, c-*fos*, c-*ras*^Ha, c-*ras*^Ki, c-*fes*, c-*myb*, and c-*src*.

15. Antibodies according to Claim 14, characterized in that they are labeled with a label capable of providing a detectable signal.

16. Antibodies according to Claim 14, characterized in that they are labeled with a cytotoxic agent.

17. An antigenic oligopeptide characterized in that it corresponds to an antigenic region in a peptide expression product of m-RNA present in a retrovinus oncogene, the antigenic oligopeptide being selected from:

(a) met-ala-phe-ala-his-asn-pro-pro-ala-gly-pro-leu-pro-gly-ala,

(b) pro-phe-his-lys-asp-gln-thr-phe-thr-glu-tyr-arg-lys-met-his-gly-gly-ala-val,

(c) pro-phe-his-lys-asp-gln-thr-phe-thr-glu-tyr-arg-lys-met,

(d) asp-asn-thr-arg-thr-ser-gly-asp-asn-ala-pro-val-ser-cys-leu-gly-glu,

(e) arg-leu-ileu-glu-asp-asn-glu-tyr-thr-ala-arg-gln-gly-ala-lys-phe-pro,

(f) arg-leu-lys-lys-ileu-ser-lys-glu-glu-lys-thr-pro-gly-cys-val-lys-ileu-lys-lys,

(g) asp-leu-pro-ser-arg-thr-val-asp-thr-lys-gln-ala-gln-glu-leu-ala-arg,

(h) met-thr-glu-tyr-lys-leu-val-val-gly-ala-ser-gly-val-gly-lys-ser-ala,

(i) glu-asp-ileu-his-gln-tyr-arg-glu-gln-ileu-lys-arg-val-lys-asp-ser-asp-asp,

(j) val-arg-glu-ileu-arg-gln-his-lys-leu-arg-lys-leu-asn-pro-pro-asp-glu-ser-gly-pro,

(k) trp-arg-arg-asp-pro-glu-glu-arg-pro-thr, and

(l) ala-ser-pro-tyr-pro-asn-leu-ser-asn-gln-thr-arg.

18. Antibodies characterized in that they are raised to an antigenic oligopeptide of Claim 17.

19. Antibodies according to Claim 18, characterized in that they are labeled with a label capable of providing a detectable signal.

20. Antibodies according to Claim 18, characterized in that they are labeled with a cytotoxic agent.

14

# 0 108 564

21. An antigenic oligopeptide characterized in that it contains up to 50 amino acid residues containing one or more of the amino acid sequences of Claim 17.

22. An antigenic oligopeptide according to Claim 21 characterized in that it contains up to 25 amino acid residues.

23. A process for preparing an antigenic oligopeptide according to any one of claims 17, 21 or 22, characterized in that it comprises sequential coupling of amino acids, in the order given by the oligopeptide formula, on a suitable support.

24. A process according to claim 23, characterized in that the support comprises a polystyrene resin support.

25. The process according to Claim 24, characterized in that the polystyrene resin support is selected from chloromethylated resins, hydroxymethyl resins and benzhydrylamine resins.

26. A process for preparing an antigenic oligopeptide according to any one of Claims 17, 21 or 22, wherein said oligopeptide contains up to 20 amino acids, characterized in that it comprises synthetically preparing a double stranded DNA sequence which codes for the amino acid sequence of the oligopeptide structure, inserting the double stranded DNA into a suitable site in a cloning vehicle or vector to form a recombinant DNA molecule and transforming an appropriate host with said recombinant DNA molecule to obtain expression of the oligopeptide.

27. For use in inducing antibody formation, an antigenic oligopeptide as claimed in claim 17.

28. Antibodies to the expression product of a gene which gene is part of a retrovirus genome capable of inducing malignancy in a normal cell of which gene is substantially complementary to said gene of said retrovirus genome, for treating a human host suspected of having malignant cells.

**Claims for the Contracting State: AT**

1. A method for evaluating the existence of cellular malignancy in a human host, said method being characterized in that it comprises:

bringing into close association (1) a probe haivng specific binding affinity for a cellular product, said cellular product being mRNA or its expression product, where said mRNA is complementary to a DNA sequence of a retrovirus capable of transforming a normal cell to malignancy, and (2) a sample from said human host suspected of containing said cellular product; and

determining the level of said cellular product by measuring the binding of said probe to said cellular product as indicative of the presence of cellular malignancy.

2. A method according to Claim 1, characterized in that said sample is cells from said human host.

3. A method according to Claim 1, characterized in that said sample is a physiological fluid from said human host.

4. A method according to Claim 1, characterized in that said DNA sequence is selected from the group consisting of the oncogenes *src, fps, yes, fos, myc, erb, myb, rel, mos, bas, abl, ras, fes, fms*, and *sis*.

5. A method according to any of Claims 1, 2, 3 or 4, characterized in that said probe is an antibody.

6. A method according to Claim 5, characterized in that said antibody is labeled with a label capable of providing a detectable signal.

7. A method according to any of Claims 1, 2, 3, or 4, characterized in that said probe is a polynucleotide of at least 14 bases complementary to said mRNA.

8. A method according to claim 1, characterized in that it comprises a method for evaluating the existence of leukemia in a human host, said method comprising:

combining a probe comprising an antibody having specific binding affinity for a cellular product of the oncogene *myb*, and blood cells from a human host suspected of having leukemia; and

determining the level of said cellular product by measuring the binding of said antibodies to said cellular product as diagnostic of a leukemic host.

9. A method according to Claim 8, characterized in that said antibodies are produced in response to an oligopeptide mimicking a portion of the conformation of the *myb* protein.

10. An *in vitro* method for substantially eliminating human malignant cells from a combination of human malignant and normal cells, characterized in that it comprises:

combining said combination of cells with an antibody specific for an expression product of a DNA sequence present in a retrovirus genome or substantially complementary to said DNA sequence, which sequence is expressed in said malignant cells as a surface protein, said combining being performed under cytotoxic conditions; and

isolating normal cells, substantially free of malignant cells.

11. A method according to Claim 10, characterized in that said separation occurs in the presence of complement as said cytotoxic condition.

12. A method according to Claim 10, characterized in that said antibodies are labeled with a radionuclide as said cytotoxic condition.

13. A method according to any of Claims 10, 11 or 12, characterized in that said DNA sequence is the myb gene.

14. A method for preparing monoclonal or polyclonal antibodies characterized in that the antibodies

15

**0 108 564**

which are prepared are specific for the expression product of the human oncogenes c-*myc*, c-*fos*, c-*ras*[Ha], c-*ras*[Ki], c-*res,* c-*myb,* and c-*src.*

15. A method according to Claim 14, characterized in that the antibodies are labeled with a label capable of providing a detectable signal.

16. A method according to Claim 14, characterized in that the antibodies are labeled with a cytotoxic agent.

17. A method for preparing an antigenic oligopeptide, characterized in that the oligopeptide which is prepared corresponds to an antigenic region in a peptide expression product of mRNA present in a retrovirous oncogene, the antigenic oligopeptide being selected from:

(a) met-ala-phe-ala-his-asn-pro-pro-ala-gly-pro-leu-pro-gly-ala,

(b) pro-phe-his-lys-asp-gln-thr-phe-thr-glu-tyr-arg-lys-met-his-gly-gly-ala-val,

(c) pro-phe-his-lys-asp-gln-thr-phe-thr-glu-tyr-arg-lys-met,

(d) asp-asn-thr-arg-thr-ser-gly-asp-asn-ala-pro-val-ser-cys-leu-gly-glu,

(e) arg-leu-ileu-glu-asp-asn-glu-tyr-thr-ala-arg-gln-gly-ala-lys-phe-pro,

(f) arg-leu-lys-lys-ileu-ser-lys-glu-glu-lys-thr-pro-gly-cys-val-lys-ileu-lys-lys,

(g) asp-leu-pro-ser-arg-thr-val-asp-thr-lys-gln-ala-gln-glu-leu-ala-arg,

(h) met-thr-glu-tyr-lys-leu-val-val-gly-ala-ser-gly-val-gly-lys-ser-ala,

(i) glu-asp-ileu-his-gln-tyr-arg-glu-gln-ileu-lys-arg-val-lys-asp-ser-asp-asp,

(j) val-arg-glu-ileu-arg-gln-his-lys-leu-arg-lys-leu-asn-pro-pro-asp-glu-ser-gly-pro,

(k) trp-arg-arg-asp-pro-glu-glu-arg-pro-thr, and

(l) ala-ser-pro-tyr-pro-asn-leu-ser-asn-gln-thr-arg.

18. A method of raising antibodies, characterized in that they are raised to an antigenic oligopeptide as defined in Claim 17.

19. A method according to Claim 18, characterized in that the antibodies are labeled with a label capable of providing a detectable signal.

20. A method according to Claim 18, characterized in that the antibodies are labeled with a cytotoxic agent.

21. A method for preparing an antigenic oligopeptide, characterized in that the oligopeptide which is prepared contains up to 50 amino acid residues containing one or more of the amino acid sequences as defined in Claim 17.

22. A method according to Claim 21 characterized in that it contains up to 25 amino acid residues.

23. A method according to any one of claims 17, 21 or 22, characterized in that it comprises sequential coupling of amino acids, in the order given by the oligopeptide formula, on a suitable support.

24. A method according to claim 23, characterized in that the support comprises a polystyrene resin support.

25. A method according to Claim 24, characterized in that the polystyrene resin support is selected from chloromethylated resins, hydroxymethyl resins and benzhydrylamine resins.

26. A method according to any one of Claims 17, 21 or 22, wherein said oligopeptide contains up to 20 amino acids, characterized in that it comprises synthetically preparing a double stranded DNA sequence which codes for the amino acid sequence of the oligopeptide structure, inserting the double stranded DNA into a suitable site in a cloning vehicle or vector to form a recombinant DNA molecule and transforming an appropriate host with said recombinant DNA molecule to obtain expression of the oligopeptide.

**Patentansprüche für die Vertragsstaaten: BE, CH, DE, FR, GB, IT, LI, LU, NL, SE**

1. Methode zur Bewertung der Existenz von zellulärer Malignität in einem menschlichen wirt, wobei dieses Verfahren dadurch gekennzeichnet ist, daß es umfaßt:

enges Assoziieren (1) einer Probe, die eine spezifische Bindungsaffinität für ein zelluläres Produkt hat, wobei das genannte zelluläre Produkt eine mRNA oder sein Expressionsprodukt ist, wobei die genannte mRNA komplementär ist zu einer DNA-Sequenz eines Retroviruses mit der Fähigkeit, eine normale Zelle zur Malignität zu transformieren, und (2) einer Probe aus dem genannten menschlichen Wirt, der verdächtigt wird, das genannte zelluläre Produkt zu enthalten; und

Bestimmen des Levels des genannten zellulären Produktes durch Messen der Bindung der genannten Probe zu dem genannten zellulären Produkt als Indikator für die Anwesenheit zellulärer Malignität.

2. Methode nach Anspruch 1, dadurch gekennzeichnet, daß die genannte Probe Zellen aus dem genannten menschlichen Wirt sind.

3. Methode nach Anspruch 1, dadurch gekennzeichnet, daß die genannte Probe eine physiologische flüssigkeit aus dem genannten menschlichen Wirt ist.

4. Methode nach Anspruch 1, dadurch gekennzeichnet, daß die genannte DNA-Sequenz ausgewählt wird aus der Gruppe umfassend die Onkogene *src, fps, yes, fos, myc, erb, myb, rel, mos, bas, abl, ras, fes,* und *sis.*

5. Methode nach einem der Ansprüche 1, 2, 3 oder 4, dadurch gekennzeichnet, daß die genannte Probe ein Antikörper ist.

6. Methode nach Anspruch 5, dadurch gekennzeichnet, daß der genannte Antikörper mit einer Markierung markiert ist, die fähig ist, ein erkennbares Signal zu liefern.

16

7. Methode nach einem der Ansprüche 1, 2, 3 oder 4, dadurch gekennzeichnet, daß die genannte Probe ein Polynukleotid mit mindestens 14 Basen komplementär zur genannten mRNA ist.

8. Methode nach Anspruch 1, dadurch gekennzeichnet, daß es ein Verfahren zur Bewertung der Existenz von Leukämie in einem menschlichen Wirt umfaßt, wobei dieses Verfahren umfaßt:

Kombinieren einer Probe enthaltend einen Antikörper, der eine spezifische Bindungsaffinität für ein zelluläres Produkt des Onkogens *myb* hat, mit Blutzellen aus einem menschlichen Wirt, der verdächtigt wird, Leukämie zu haben; und

Bestimmen des levels des genannten zellulären Produktes durch Messen der Bindung der genannten Antikörper an das genannte zelluläre Produkt als Diagnosemittel für einen Leukämiepatienten.

9. Methode nach Anspruch 8, dadurch gekennzeichnet, daß die genannten Antikörper als Antwort auf ein Oligopeptid produziert werden, das einen Teil der Konformation des *myb* Proteins nachahmt.

10. In vitro Methode zur substantiellen Eliminierung maligner menschlicher Zellen aus einer Kombination von malignen und normalen menschlichen Zellen, dadurch gekennzeichnet, daß es umfaßt:

Kombinieren der genannten Kombination von Zellen mit einem Antikörper, der spezifisch für ein Expressionsprodukt einer DNA-Sequenz ist, die in einem Retrovirusgenom anwesend oder substantiell komplementär zur genannten DNA-Sequenz ist, wobei diese Sequenz in den genannten malignen Zellen als ein Oberflächenprotein exprimiert wird, wobei das genannte Kombinieren unter zytotoxischen Bedingungen durchgeführt wird; und

isolieren von normalen Zellen, im wesentlichen frei von malignen Zellen.

11. Methode nach Anspruch 10, dadurch gekennzeichnet, daß die Trennung in Gegenwart von Komplement als die genannte zytotoxische Bedingung erfolgt.

12. Methode nach Anspruch 10, dadurch gekennzeichnet, daß die genannten Antikörper mit einem Radionuklid als die genannte zytotoxische Bedingung markiert werden.

13. Methode nach einem der Ansprüche 10, 11 oder 12, dadurch gekennzeichnet, daß die DNA-Sequenz das myb Gen ist.

14. Antikörper, dadurch gekennzeichnet, daß sie spezifisch sind für das Expressionsprodukt der menschlichen Onkogene c-*myc*, c-*fos*, c-*ras*^Ha, c-*ras*^Kl, c-*fes*, c-*myb*, und c-*src*.

15. Antikörper nach Anspruch 14, dadurch gekennzeichnet, daß sie mit einer Markierung markiert sind, die fähig ist, ein erkennbares Signal zu liefern.

16. Antikörper nach Anspruch 14, dadurch gekennzeichnet, daß sie mit einem zytotoxischen Agens markiert sind.

17. Antigenes Oligopeptid, dadurch gekennzeichnet, daß es korrespondiert mit einer antigenen Region in einem Peptid, dem Expressionsprodukt einer mRNA, das in einem Retrovirusonkogen enthalten ist, wobei das antigene Oligopeptid ausgewählt wird aus:

(a) met-ala-phe-ala-his-asn-pro-pro-ala-gly-pro-leu-pro-gly-ala,

(b) pro-phe-his-lys-asp-gln-thr-phe-thr-glu-tyr-arg-lys-met-his-gly-gly-ala-val,

(c) pro-phe-his-lys-asp-gln-thr-phe-thr-glu-tyr-arg-lys-met,

(d) asp-asn-thr-arg-thr-ser-gly-asp-asp-ala-pro-val-ser-cys-leu-gly-glu,

(e) arg-leu-ileu-glu-asp-asn-glu-tyr-thr-ala-arg-gln-gly-ala-lys-phe-pro,

(f) arg-leu-lys-lys-ileu-ser-lys-glu-glu-lys-thr-pro-gly-cys-val-lys-ileu-lys-lys,

(g) asp-leu-pro-ser-arg-thr-val-asp-thr-lys-gln-ala-gln-glu-leu-ala-arg,

(h) met-thr-glu-tyr-lys-leu-val-val-gly-ala-ser-gly-val-gly-lys-ser-ala,

(i) glu-asp-ileu-his-gln-tyr-arg-glu-gln-ileu-lys-arg-val-lys-asp-ser-asp-asp,

(j) val-arg-glu-ileu-arg-gln-his-lys-leu-arg-lys-leu-asn-pro-pro-asp-glu-ser-gly-pro,

(k) trp-arg-arg-asp-pro-glu-glu-arg-pro-thr, und

(l) ala-ser-pro-tyr-pro-asn-leu-ser-asn-gln-thr-arg.

18. Antikörper, dadurch gekennzeichnet, daß sie gegen ein antigenes Oligopeptid des Anspruches 17 erzeugt werden.

19. Antikörper nach Anspruch 18, dadurch gekennzeichnet, daß sie mit einer Markierung markiert sind, die fähig ist, ein erkennbares Signal zu liefern.

20. Antikörper nach Anspruch 18, dadurch gekennzeichnet, daß sie mit einem zytotoxischen Agens markiert sind.

21. Antigenes Oligopeptid, dadurch gekennzeichnet, daß es bis zu 50 Aminosäurereste enthält, die eine oder mehrere der Aminosäure-Sequenzen des Anspruches 17 enthalten.

22. Antigenes Oligopeptid nach Anspruch 21, dadurch gekennzeichnet, daß es bis zu 25 Aminosäurereste enthält.

23. Verfahren zur Herstellung eines antigenen Oligopeptids nach einem der Ansprüche 17, 21 oder 22, dadurch gekennzeichnet, daß es sequentielles Koppeln von Aminosäuren, in der durch die Oligopeptidformel gegebenen Reihenfolge, auf einem geeigneten Träger umfaßt.

24. Verfahren nach Anspruch 23, dadurch gekennzeichnet, daß der Träger einen Polystyrolhärzträger umfaßt.

25. Verfahren nach Anspruch 24, dadurch gekennzeichnet, daß der Polystyrolharzträger aus chlormethylierten Harzen, Hydroxymethylharzen und Benzhydrylaminharzen ausgewählt wird.

26. Verfahren zur Herstellung eines antigenen Oligopeptids nach einem der Ansprüche 17, 21 und 22, worin das genannte Oligopeptid bis zu 20 Aminosäuren enthält, dadurch gekennzeichnet, daß es umfaßt:

## 0 108 564

synthetisches Herstellen einer doppelsträngigen DNA-Sequenz, die für die Aminosäure-Sequenz der Oligopeptidstruktur kodiert, Inserieren der doppelsträngigen DNA an einer geeigneten Stelle in ein Klonierungsvehikel oder einen Klonierungsvektor, um ein rekombinantes DNA-Molekül zu bilden, und Transformieren eines passenden Wirtes mit dem genannten DNA-Molekül, um eine Expression des Oligopeptids zu erhalten.

27. Zur Verwendung zum Induzieren einer Antikörperbildung, ein antigenes Oligopeptid nach Anspruch 17.

28. Antikörper gegen das Expressionsprodukt eines Gens, wobei dieses Gen Teil eines Retrovirusgenoms ist, das fähig ist, Malignität in eine normale Zelle zu induzieren, oder wobei dieses Gen substantiell komplementär zu dem genannten Gen des genannten Retrovirusgenoms ist, zur Behandlung eines menschlichen Wirtes, der verdächtigt wird, maligne Zellen zu haben.

**Patentansprüche für den Vertragsstaat: AT**

1. Verfahren zur Bewertung der Existenz von zellulärer Malignität in einem menschlichen Wirt, wobei dieses verfahren dadurch gekennzeichnet ist, daß es umfaßt:

enges Assoziieren (1) einer Probe, die eine spezifische Bindungsafinität für ein zelluläres Produkt hat, wobei das genannte zelluläre Produkt eine mRNA oder sein Expressionsprodukt ist, wobei die genannte mRNA komplementär ist zu einer DNA-Sequenz eines Retroviruses mit der Fähigkeit, eine normale Zelle zur Malignität zu transformieren, und (2) einer Probe aus dem genannten menschlichen Wirt, der verdächtigt wird, das genannte zelluläre Produkt zu enthalten; und

Bestimmen des Levels des genannten zellulären Produktes durch Messen der Bindung der genannten Probe zu dem genannten zellulären Produkt als Indikator für die Anwesenheit zellulärer Malignität.

2. Verfahren nach Anspruch 1, dadurch gekennzeichnet, daß die genannte Probe Zellen aus dem genannten menschlichen Wirt sind.

3. Verfahren nach Anspruch 1, dadurch gekennzeichnet, daß die genannte Probe eine physiologische Flüssigkeit aus dem genannten menschlichen Wirt ist.

4. Verfahren nach Anspruch 1, dadurch gekennzeichnet, daß die genannte DNA-Sequenz ausgewählt wird aus der Gruppe umfassend die Onkogene *src, fps, yes, fos, myc, erb, myb, rel, mos, bas, abl, ras, fes,* und *sis.*

5. Verfahren nach einem der Ansprüche 1, 2, 3 oder 4, dadurch gekennzeichnet, daß die genannte Probe ein Antikörper ist.

6. Verfahren nach Anspruch 5, dadurch gekennzeichnet, daß der genannte Antikörper mit einer Markierung markiert ist, die fähig ist, ein erkennbares Signal zu liefern.

7. Verfahren nach einem der Ansprüche 1, 2, 3 oder 4, dadurch gekennzeichnet, daß die genannte Probe ein Polynukleotid mit mindestens 14 Basen komplementar zur genannten mRNA ist.

8. Verfahren nach Anspruch 1, dadurch gekennzeichnet, daß es ein Verfahren zur Berwertung der Existenz von Leukämie in einem menschlichen Wirt umfaßt, wobei dieses Verfahren umfaßt:

Kombinieren einer Probe enthaltend einen Antikörper, der eine spezifische Bindungsaffinität für ein zelluläres Produkt des Onkogens *myb* hat, mit Blutzellen aus einem menschlichen Wirt, der verdächtigt wird, Leukämie zu haben; und

Bestimmen des Levels des genannten zellulären Produktes durch Messen der Bindung der genannten Antikörper an das genannte zelluläre Produkt als Diagnosemittel für einen Leukämiepatienten.

9. Verfahren nach Anspruch 8, dadurch gekennzeichnet, daß die genannten Antikörper als Antowrt auf ein Oligopeptid produziert werden, das einen Teil der Konformation des *myb* Proteins nachahmt.

10. In vitro Verfahren zur substantiellen Eliminierung maligner menschlicher Zellen aus einer Kombination von malignen und normalen menschlichen Zellen, dadurch gekennzeichnet, daß es umfaßt:

Kombinieren der genannten Kombination von Zellen mit einem Antikörper, der spezifisch für ein Expressionsprodukt einer DNA-Sequenz ist, die in einem Retrovirusgenom anwesend oder substantiell komplementär zur genannten DNA-Sequenz ist, wobei diese Sequenz in den genannten malignen Zellen als ein Oberflächenprotein exprimiert wird, wobei das genannte Kombinieren unter zytotoxischen Bedingungen durchgeführt wird; und

Isolieren von normalen Zellen, im wesentlichen frei von malignen Zellen.

11. Verfahren nach Anspruch 10, dadurch gekennzeichnet, daß die Trennung in Gegenwart von Komplement als die genannte zytotoxische Bedingung erfolgt.

12. Verfahren nach Anspruch 10, dadurch gekennzeichnet, daß die genannten Antikörper mit einem Radionuklid als die genannte zytotoxische Bedingung markiert werden.

13. Verfahren nach einem der Ansrpüche 10, 11 oder 12, dadurch gekennzeichnet, daß die DNA-Sequenz das myb Gen ist.

14. Verfahren zur Herstellung von monoklonalen und polyklonalen Antikörpern, dadurch gekennzeichnet, daß die Antikörper, die hergestellt werden, spezifisch sind für das Expressionsprodukt der menschlichen Onkogene c-*myc*, c-*fos*, c-*ras*$^{Ha}$, c-*ras*$^{Kl}$, c-*fes*, c-*myb*, und c-*src*.

15. Verfahren nach Anspruch 14, dadurch gekennzeichnet, daß die Antikörper mit einer Markierung markiert sind, die fähig ist, ein erkennbares Signal zu liefern.

18

16. Verfahren nach Anspruch 14, dadurch gekennzeichnet, daß die Antikörper mit einem zytotoxischen Agens markiert sind.

17. Verfahren zur Herstellung eines antigenen Oligopeptids, dadurch gekennzeichnet, daß das Oligopeptid, das hergestellt wird, korrespondiert mit einer antigenen Region in einem Peptid, dem Expressionsprodukt einer mRNA, das in einem Retrovirusonkogen enthalten ist, wobei das antigene Oligopeptid ausgewählt wird aus:

(a) met-ala-phe-ala-his-asn-pro-pro-ala-gly-pro-leu-pro-gly-ala,

(b) pro-phe-his-lys-asp-gln-thr-phe-thr-glu-tyr-arg-lys-met-his-gly-gly-ala-val,

(c) pro-phe-his-lys-asp-gln-thr-phr-thr-glu-tyr-arg-lys-met,

(d) asp-asn-thr-arg-thr-ser-gly-asp-asn-ala-pro-val-ser-cys-leu-gly-glu,

(e) arg-leu-ileu-glu-asp-asn-glu-tyr-thr-ala-arg-gln-gly-ala-lys-phe-pro,

(f) arg-leu-lys-lys-ileu-ser-lys-glu-glu-lys-thr-pro-gly-cys-val-lys-ileu-lys-lys,

(g) asp-leu-pro-ser-arg-thr-val-asp-thr-lys-gln-ala-gln-glu-leu-ala-arg,

(h) met-thr-glu-tyr-lys-leu-val-val-gly-ala-ser-gly-val-gly-lys-ser-ala,

(i) glu-asp-ileu-his-gln-tyr-arg-glu-gln-ileu-lys-arg-val-lys-asp-ser-asp-asp,

(j) val-arg-glu-ileu-arg-gln-his-lys-leu-arg-lys-leu-asn-pro-pro-asp-glu-ser-gly-pro,

(k) trp-arg-arg-asp-pro-glu-glu-arg-pro-thr, und

(l) ala-ser-pro-tyr-pro-asn-leu-ser-asn-gln-thr-arg.

18. Verfahren zur Erzeugung von Antikörpen, dadurch gekennzeichnet, daß sie gegen ein antigenes Oligopeptid, wie in Anspruch 17 definiert, erzeugt werden.

19. Verfahren nach Anspruch 18, dadurch gekennzeichnet, daß die Antikörper mit einer Markierung markiert sind, die fähig ist, ein erkennbares Signal zu liefern.

20. Verfahren nach Anspruch 18, dadurch gekennzeichnet, daß die Antikörper mit einem zytotoxischen Agens markiert sind.

21. Verfahren zur Herstellung eines antigenen Oligopeptids, dadurch gekennzeichnet, daß das Oligopeptid, das hergestellt wird, bis zu 50 Aminosäurereste enthält, die eine oder mehrere der Aminosäure-Sequenzen, wie in Anspruch 17 definiert, enthälten.

22. Verfahren nach Anspruch 21, dadurch gekennzeichnet, daß es bis zu 25 Aminosäurereste enthält.

23. Verfahren nach einem der Ansprüche 17, 21 oder 22, dadurch gekennzeichnet, daß es sequentielles Koppeln von Aminosäuren, in der durch die Oligopeptidformel gegebenen Reihenfolge, auf einem geeigneten Träger umfaßt.

24. Verfahren nach Anspruch 23, dadurch gekennzeichnet, daß der Träger einen Polystyrolharzträger umfaßt.

25. Verfahren nach Anspruch 24, dadurch gekennzeichnet, daß der Polystyrolharzträger aus chlormethylierten Harzen, Hydroxymethylharzen und Benzhydrylaminharzen ausgewählt wird.

26. Verfahren zur Herstellung eines antigenen Oligopeptids nach einem der Ansprüche 17, 21 und 22, worin das genannte Oligopeptid bis zu 20 Aminosäuren enthält, dadurch gekennzeichnet, daß es umfaßt: synthetisches Herstellen einer doppelsträngigen DNA-Sequenz, die für die Aminosäure-Sequenz der Oligopeptidstruktur kodiert, Inserieren der doppelsträngigen DNA an einer geeigneten Stelle in ein Klonierungsvehikel oder einen Klonierungsvektor, um ein rekombinantes DNA-Molekül zu bilden, und Transformieren eines passenden Wirtes mit dem genannten DNA-Molekül, um eine Expression des Oligopeptids zu erhalten.

**Revendications pour les Etats Contractants: BE, CH, DE, FR, GB, IT, LI, LU, NL, SE**

1. Un procédé pour l'évaluation de l'existence d'une malignité cellulaire chez un hôte humain, ledit procédé étant caractérisé en ce qu'il consiste à:

mettre en association étroite (1) une sonde ayant une affinité de liaison spécifique pour un produit cellulaire, ledit produit cellulaire étant un ARNm ou son produit d'expression, ledit ARNm étant complémentaire d'une séquence d'ADN d'un rétrovirus capable de transformer une cellule normale en la rendant maligne et (2) un échantillon dudit hôte humain suspecté de contenir ledit produit cellulaire; et

déterminer le taux dudit produit cellulaire par mesure de la liaison de ladite sonde audit produit cellulaire comme indication de la présence d'une malignité cellulaire.

2. Un procédé selon la revendication 1, caractérisé en ce que ledit échantillon consiste en des cellules dudit hôte humain.

3. Un procédé selon la revendication 1, caractérisé en ce que ledit échantillon est un liquide physiologique dudit hôte humain.

4. Un procédé selon la revendication 1, caractérisé en ce que ladite séquence d'ADN est choisie dans le groupe constitué par les oncogènes *src, fps, yes, fos, myc, erb, myb, rel, mos, bas, abl, ras, fes, fms* et *sis*.

5. Un procédé selon l'une quelconque des revendications 1, 2, 3 ou 4, caractérisé en ce que ladite sonde est un anticorps.

6. Un procédé selon la revendication 5, caractérisé en ce que ledit anticorps est marqué avec un marqueur capable de fournir un signal détectable.

7. Un procédé selon l'une quelconque des revendications 1, 2, 3 ou 4, caractérisé en ce que ladite sonde est un polynucléotide d'au moins 14 bases complémentaire dudit ARNm.

## 0 108 564

8. Un procédé selon la revendication 1, caractérisé en ce qu'il comprend un procédé pour évaluer l'existence d'une leucémie chez un hôte humain, ledit procédé consistant à:

combiner une sonde comprenant un anticorps ayant une affinité de liaison spécifique pour un produit cellulaire de l'oncogène *myb* et des cellules sanguines d'un hôte humain soupçonné d'être atteint de leucémie; et

déterminer le taux dudit produit cellulaire par mesure de la liaison desdits anticorps audit produit cellulaire comme diagnostic d'un hôte leucémique.

9. Un procédé selon la revendication 8, caractérisé en ce que lesdits anticorps sont produits en réponse à un oligopeptide imitant une portion de la conformation de la protéine *myb*.

10. Un procédé in vitro pour éliminer essentiellement les cellules malignes humaines d'une combinaison de cellules malignes humaines et de cellules normales humaines, caractérisé en ce qu'il consiste à:

combiner ladite combinaison de cellules avec un anticorps spécifique d'un produit d'expression d'une séquence d'ADN présente dans un génome de rétrovirus ou essentiellement complémentaire de ladite séquence d'ADN, laquelle séquence est exprimée dans lesdites cellules malignes comme protéine de surface, ladite combinaison étant effectuée dans des conditions cytotoxiques; et

isoler les cellules normales essentiellement débarrassées des cellules malignes.

11. Un procédé selon la revendication 10, caractérisé en ce que ladite séparation se produit en présence de complément comme dite condition cytotoxique.

12. Un procédé selon la revendication 10, caractérisé en ce que lesdits anticorps sont marqués avec un radionucléide comme dite condition cytotoxique.

13. Un procédé selon l'une quelconque des revendications 10, 11 ou 12, caractérisé en ce que ladite séquence d'ADN est le gène *myb*.

14. Anticorps caractérisés en ce qu'ils sont spécifiques du produit d'expression des oncogènes humains c-*myc*, c-*fos*, c-*ras*$^{Ha}$, c-*ras*$^{Kl}$, c-*fes*, c-*myb* et c-*src*.

15. Anticorps selon la revendication 14, caractérisés en ce qu'ils sont marqués avec un marqueur capable de fournir un signal détectable.

16. Anticorps selon la revendication 14, caractérisés en ce qu'ils sont marqués avec un agent cytotoxique.

17. Un oligopeptide antigénique, caractérisé en ce qu'il correspond à une région antigénique d'un produit d'expression peptidique de ARNm présent dans un oncogène rétroviral, l'oligopeptide antigénique étant choisi parmi:

(a) met-ala-phe-ala-his-asn-pro-pro-ala-gly-pro-leu-pro-gly-ala

(b) pro-phe-his-lys-asp-gln-thr-phe-thr-glu-tyr-arg-lys-met-his-gly-gly-ala-val

(c) pro-phe-his-lys-asp-gln-thr-phe-thr-glu-tyr-arg-lys-met

(d) asp-asn-thr-arg-thr-ser-gly-asp-asn-ala-pro-val-ser-cys-leu-gly-glu

(e) arg-leu-ileu-glu-asp-asn-glu-tyr-thr-ala-arg-gln-gly-ala-lys-phe-pro

(f) arg-leu-lys-lys-ileu-ser-lys-glu-glu-lys-thr-pro-gly-cys-val-lys-ileu-lys-lys

(g) asp-leu-pro-ser-arg-thr-val-asp-thr-lys-gln-ala-gln-glu-leu-ala-arg

(h) met-thr-glu-tyr-lys-leu-val-val-gly-ala-ser-gly-val-gly-lys-ser-ala

(i) glu-asp-ileu-his-gln-tyr-arg-glu-gln-ileu-lys-arg-val-lys-asp-ser-asp-asp

(j) val-arg-glu-ileu-arg-gln-his-lys-leu-arg-lys-leu-asn-pro-pro-asp-glu-ser-gly-pro

(k) trp-arg-arg-asp-pro-glu-glu-arg-pro-thr, et

(l) ala-ser-pro-tyr-pro-asn-leu-ser-asn-glu-thr-arg.

18. Anticorps caractérisés en ce qu'ils ont été induits contre un oligopeptide antigénique de la revendication 17.

19. Anticorps selon la revendication 18, caractérisés en ce qu'ils sont maqués avec un marqueur capable de fournir un signal détectable.

20. Anticorps selon la revendication 18, caractérisés en ce qu'ils sont marqués avec un agent cytotoxique.

21. Un oligopeptide antigénique, caractérisé en ce qu'il contient jusqu'à 50 restes d'amino-acides contenant une ou plusieurs des séquences d'amino-acides de la revendication 17.

22. Un oligopeptide antigénique selon la revendication 21, caractérisé en ce qu'il contient jusqu'à 25 restes d'amino-acides.

23. Un procédé pour préparer un oligopeptide antigénique selon l'une quelconque des revendications 17, 21 ou 22, caractérisé en ce qu'il comprend le couplage successif d'amino-acides, dans l'ordre indiqué par la formule de l'oligopeptide, sur un support approprié.

24. Un procédé selon la revendication 23, caractérisé en ce que le support est constitué d'un support de résine de polystyrène.

25. Le procédé selon la revendication 24, caractérisé en ce que le support de résine de polystyrène est choisi parmi les résines chlorométhylées, les résines hydroxyméthylées et les résines à benzhydrylamine.

26. Un procédé de préparation d'un oligopeptide antigenique selon l'une quelconque des revendications 17, 21 ou 22, dans lequel ledit oligopeptide contient jusqu'à 20 amino-acides, caractérisé en ce qu'il comprend la préparation synthétique d'une séquence d'ADN bicaténaire qui code pour la séquence d'amino-acides de la structure de l'oligopeptide, l'insertion de l'ADN bicaténaire en un site approprié dans

20

## 0 108 564

un véhicule ou vecteur de clonage pour former une molécule d'ADN recombinant et la transformation d'un hôte approprié avec ladite molécule d'ADN recombinant pour obtenir l'expression de l'oligopeptide.

27. Un oligopeptide antigénique selon la revendication 17 pour l'emploi dans l'induction de la formation d'anticorps.

28. Anticorps centre le produit d'expression d'un gène, lequel gène fait partie d'un génome rétroviral capable d'induire la malignité d'une cellule normale ou lequel gène est essentiellement complémentaire dudit gène dudit génome rétroviral, pour le traitement d'un hôte humain soupçonné d'avoir des cellules malignes.

**Revendications pou l'Etat Contractant: AT**

1. Un procédé pour l'évaluation de l'existence d'une malignité cellulaire chez un hôte humain, ledit procédé étant caractérisé en ce qu'il consiste à:

mettre en association étroite (1) une sonde ayant une affinité de liaison spécifique pour un produit cellulaire, ledit produit cellulaire étant un ARNm ou son produit d'expression, ledit ARNm étant complémentaire d'une séquence d'ADN d'un rétrovirus capable de transformer une cellule normale en la rendant maligne et (2) un échantillon dudit hôte humain suspecté de contenir ledit produit cellulaire; et

déterminer le taux dudit produit cellulaire par mesure de la liaison de ladite sonde audit produit cellulaire comme indication de la présence d'une malignité cellulaire.

2. Un procédé selon la revendication 1, caractérisé en ce que ledit échantillon consiste en des cellules dudit hôte humain.

3. Un procédé selon la revendication 1, caractérisé en ce que ledit échantillon est un liquide physiologique dudit hôte humain.

4. Un procédé selon la revendication 1, caractérisé en ce que ladite séquence d'ADN est choisie dans le groupe constitué par les oncogènes *src, fps, yes, fos, myc, erb, myb, rel, mos, bas, abl, ras, fes, fms* et *sis.*

5. Un procédé selon l'une quelconque des revendications 1, 2, 3 ou 4, caractérisé en ce que ladite sonde est un anticorps.

6. Un procédé selon la revendication 5, caractérisé en ce que ledit anticorps est marqué avec un marqueur capable de fournir un signal détectable.

7. Un procédé selon l'une quelconque des revendications 1, 2, 3 ou 4, caractérisé en ce que ladite sonde est un polynucléotide d'au moins 14 bases complémentaire dudit ARNm.

8. Un procédé selon la revendication 1, caractérisé en ce qu'il comprend un procédé pour évaluer l'existence d'une leucémie chez un hôte humain, ledit procédé consistant à:

combiner une sonde comprenant un anticorps ayant une affinité de liaison spécifique pour un produit cellulaire de l'oncogène *myb* et des cellules sanguines d'un hôte humain soupçonné d'être atteint de leucémie; et

déterminer le taux dudit produit cellulaire par mesure de la liaison desdits anticorps audit produit cellulaire comme diagnostic d'un hôte leucémique.

9. Un procédé selon la revendication 8, caractérisé en ce que lesdits anticorps sont produits en réponse à un oligopeptide imitant une portion de la conformation de la protéine *myb.*

10. un procédé in vitro pour éliminer essentiellement les cellules malignes humaines d'une combinaison de cellules malignes humaines et de cellules normales humaines, caractérisé en ce qu'il consiste à:

combiner ladite combinaison de cellules avec un anticorps spécifique d'un produit d'expression d'une séquence d'ADN présente dans un génome de rétrovirus ou essentiellement complémentaire de ladite séquence d'ADN, laquelle séquence est exprimée dans lesdites cellules malignes comme protéine de surface, ladite combinaison étant effectuée dans des conditions cytotoxiques; et

isoler les cellules normales essentiellement débarrassées des cellules malignes.

11. Un procédé selon la revendication 10, caractérisé en ce que ladite séparation se produit en présence de complément comme dite condition cytotoxique.

12. Un procédé selon la revendication 10, caractérisé en ce que lesdits anticorps sont marqués avec un radionucléide comme dite condition cytotoxique.

13. Un procédé selon l'une quelconque des revendications 10, 11 ou 12, caractérisé en ce que ladite séquence d'ADN est le gène *myb.*

14. Un procédé de préparation d'anticorps monoclonaux ou polyclonaux, caractérisé en ce que les anticorps qui sont préparés sont spécifiques du produit d'expression des oncogènes humains c-*myc*, c-*fos*, c-*ras*$^{Ha}$, c-*ras*$^{Kl}$, c-*fes*, c-*myb* et c-*src.*

15. Un procédé selon la revendication 14, caractérisé en ce que les anticorps sont marqués avec un marqueur capable de fournir un signal détectable.

16. Un procédé selon la revendication 14, caractérisé en ce que les anticorps sont marqués avec un agent cytotoxique.

17. Un procédé de préparation d'un oligopeptide antigénique, caractérisé en ce que l'oligopeptide qui est préparé correspond à une région antigénique d'un produit d'expression peptidique de ARNm présent dans un oncogène rétroviral, l'oligopeptide antigénique étant choisi parmi:

21

(a) met-ala-phe-ala-his-asn-pro-pro-ala-gly-pro-leu-pro-gly-ala
(b) pro-phe-his-lys-asp-gln-thr-phe-thr-glu-tyr-arg-lys-met-his-gly-gly-ala-val
(c) pro-phe-his lys-asp-gln-thr-phe-thr-glu-tyr-arg-lys-met
(d) asp-asn-thr-arg-thr-ser-gly-asp-asn-ala-pro-val-ser-cys-leu-gly-glu
(e) arg-leu-ileu-glu-asp-asn-glu-tyr-thr-ala-arg-gln-gly-ala-lys-phe-pro
(f) arg-leu-lys-lys-ileu-ser-lys-glu-glu-lys-thr-pro-gly-cys-val-lys-ileu-lys-lys
(g) asp-leu-pro-ser-arg-thr-val-asp-thr-lys-gln-ala-gln-glu-leu-ala-arg
(h) met-thr-glu-tyr-lys-leu-val-val-gly-ala-ser-gly-val-gly-lys-ser-ala
(i) glu-asp-ileu-his-gln-tyr-arg-glu-gln-ileu-lys-arg-val-lys-asp-ser-asp-asp
(j) val-arg-glu-ileu-arg-glu-his-lys-leu-arg-lys-leu-asn-pro-pro-asp-glu-ser-gly-pro
(k) trp-arg-asp-pro-glu-glu-arg-pro-thr, et
(l) ala-ser-pro-tyr-pro-asn-leu-ser-asn-gln-thr-arg.

18. Un procédé pour induire des anticorps, caractérisé en ce qu'ils sont induits contre un oligopeptide antigénique comme défini dans la revendication 17.

19. Un procédé selon la revendication 18, caractérisé en ce que les anticorps sont marqués avec un marqueur capable de fournir un signal détectable.

20. Un procédé selon la revendication 18, caractérisé en ce que les anticorps sont marqués avec un agent cytotoxique.

21. Un procédé de préparation d'un oligopeptide antigenique, caractérisé en ce que l'oligopeptide qui est préparé contient jusqu'à 50 restes d'amino-acides contenant une ou plusieurs des séquences d'amino-acides comme défini dans la revendication 17.

22. Un procédé selon la revendication 21, caractérisé en ce qu'il contient jusqu'à 25 restes d'amino-acides.

23. Un procédé selon l'une quelconque des revendications 17, 21 ou 22, caractérisé en ce qu'il comprend le couplage successif d'amino-acides dans l'ordre indiqué par la formule de l'oligopeptide, sur un support approprié.

24. Un procédé selon la revendication 23, caractérisé en ce que le support est constitué d'un support de résine de polystyrene.

25. Le procédé selon la revendication 24, caractérisé en ce que le support de résine de polystyrène est choisi parmi les résines chlorométhylées les résines hydroxyméthylées et les résines à benzhydrylamine.

26. Un procédé de préparation d'un oligopeptide antigénique selon l'une quelconque des revendications 17, 21 ou 22, dans lequel ledit oligopeptide contient jusqu'à 20 amino-acides, caractérisé en ce qu'il comprend la préparation synthétique d'une séquence d'ADN bicaténaire qui code pour la séquence d'amino-acides de la structure de l'oligopeptide, l'insertion de l'ADN bicaténaire en un site approprié dans un véhicule ou vecteur de clonage pour former une molécule d'ADN recombinant et la transformation d'un hôte approprié avec ladite molécule d'ADN recombinant pour obenir l'expression de l'oligopeptide.